# EUROPEAN PATENT SPECIFICATION

(11) **EP 0 690 054 B1**
(45) Date of publication and mention of the grant of the patent: **20.11.2002**
(21) Application number: 95110294.6
(22) Date of filing: 30.06.1995
(51) Int. Cl.: C07D 333/54, C07D 307/79, C07D 209/88

(54) **1-Amino-2-cyclohexene derivatives and production process therefor**
1-Amino-2-Cyclohexenderivate und Verfahren zu ihrer Herstellung
Dérivés d'1-amino-2-cyclohexène et procédé pour leur préparation

(30) Priority: 30.06.1994 JP 14959794
(43) Date of publication of application: 03.01.1996
(73) Proprietor: KURARAY CO., LTD., Kurashiki-City (JP)
(72) Inventor: Nakagawa, Naoshi, c/o Kuraray Co., Ltd., Okayama-ken, 710 (JP); Hatanaka, Tadashi, c/o Kuraray Co., Ltd., Okayama-ken, 710 (JP); Hayashibara, Tatsuhiko, c/o Kuraray Co., Ltd., Okayama-ken, 710 (JP); Shiono, Manzo, c/o Kuraray Co., Ltd., Okayama-ken, 710 (JP)
(74) Representative: VOSSIUS & PARTNER

(56) References cited:
- EP-A- 0 240 107
- J. CHEM. SOC. CHEM. COMM., no. 21, 1989 pages 1692-1693, S.V. KESSAR ET AL
- CHEMICAL ABSTRACTS, vol. 111, no. 8, 1989 Columbus, Ohio, US; abstract no. 69697k, & AN. QUIM., SER. B, vol. 84, no. 3, 1988 pages 279-286, A. MACIAS ET AL & DATABASE REGISTRY CHEMICAL ABSTRACTS SERVICE, COLUMBUS, OHIO, US (STN),
- 'BEILSTEIN HANDBOOK OF ORGANIC CHEMISTRY, 4th Ed., 5th Supplementary Series, Vol. 17, Part 9' , SPRINGER-VERLAG , BERLIN, DE * page 401 *

## Description

The present invention concerns 1-amino-2-cyclohexene derivatives and a production process therefor. The 1-amino-2-cyclohexene derivatives provided by the present invention are useful as an intermediate product for various kinds of medicines and agricultural agents.

It has been found in recent years that condensed ring compounds containing hetero atoms (such as an oxygen atom, a nitrogen atom and a sulfur atom) have various biological activities and vigorous research has been carried out to develop them as medicines and agricultural agents. For example, Amemiya et al. have reported that a dihydrobenzothiophene derivative or a tetrahydrobenzothiophene derivative has an inhibitory action on thromboxane synthetase (Journal of Medicinal Chemistry, 1989, vol. 32, pp. 1265-1272 and EP-A 240107), and Nagai et al. have reported that a carbazole-3,4-dicarboximide derivative has anti-tumor activity (Japanese Patent Laid-Open No. 4-178387). Further, Dubroeucq et al. have reported that a benzofuran or a benzothiophene carboxamide has an effect as a tranquilizer, anti-anginal drug and immunomodulator (Japanese Patent Laid-Open No. 63-39874).

Various studies have been made also for production processes for the condensed ring compounds. Generally, for condensed ring forming reactions, a method of bonding side chains of cyclic compounds having two side chains with each other has often been used as represented by the Robinson anellation. For instance in the report of Amemiya et al., 4,5-dihydrobenzo[b]thiophene-6-carboxylic acid methyl ester is obtained by forming 7-oxo-4,5,6,7-tetrahydrobenzo[b]thiophene using 3-bromothiophene as a starting material (MacDowell et al., Journal of Heterocyclic Chemistry, 1965, vol. 2, pp. 44-48), methoxycarbonylating the same, reducing the ketone and then dehydrating with an acid. Further, in the method of Nagai et al., a carbazole skeleton is formed by Fisher's method of indole synthesis using N-benzyl-4-oxocyclohexane-1,2-carboximide as the starting material and then reacting the same with phenylhydrazine. Further, in Dubroeucq et al., 4-phenylbenzo[b]thiophene-6-carboxylic acid is obtained by condensating 3-benzoylpropionic acid and 2-thiophene carboxy aldehyde in the presence of acetic anhydride and potassium acetate to obtain 5-phenyl-3-(2-thienylmethylene)-2-furanone and heating the same in acetic acid in the presence of methane sulfonic acid. Further, Kido et al. reported a method of constructing a 2,4,5,6,7,7a-hexahydrobenzofuran-2-one skeleton by a condensation reaction of 2-methyl-3-vinylbutenolide and 2-formyl-6-methyl-5-heptenic acid methyl ester and then introducing the same into furoventalene having a benzofuran skeleton isolated from sea fan (*Gorgonia ventalina*) (Journal of Organic Chemistry, 1981, vol. 46, pp. 4264-4266).

As described above, condensed ring compounds are useful as intermediate products for the synthesis of various medicines and agricultural agents and it is considered that they are important also for the future development of novel pharmaceutical and agricultural agents and production processes applicable to various derivatives have been demanded. However, it is still one of those subjects wherein few general production processes are available. Development for such general production processes are in demand at present. The method as described above of bonding side chains of cyclic compounds having two side chains with each other often requires a multitude of steps for the cyclizing reaction and, accordingly, functional groups that can be introduced may sometimes be restricted depending on the reaction conditions. In addition, it is also unsatisfactory in that it cannot always be used as a general process for producing various condensed ring compounds in view of the lack of availability of the starting materials.

On the other hand, a cycloaddition reaction typically represented by the Diels-Alder reaction has the feature of being capable of forming a condensed ring in a single stage since two bonds can be formed at once. As application examples, there are known, for example, indole alkaloid synthesis by way of an indole quinodimethane type diene (Magnus et al., Tetrahedron, 1981, vol, 37, pp. 3889-3897; Journal of American Chemical Society, 1982, vol. 104, pp. 1140-1141; Journal of American Chemical Society, 1983, vol. 105, pp. 4739-4749; Journal of American Chemical Society, 1983, vol. 105, pp. 4750-4757; Journal of American Chemical Society, 1984, vol. 106, pp. 2105-2114 and Accounts of Chemical Research, 1984, vol. 17, pp. 35-41), carbazole synthesis using pyrano[3,4-b]indol-3-one or pyrano[4,3-b]indol-3-one (Doren et al., Tetrahedron, 1989, vol. 45, pp. 6761-6770; Moody et al., Journal of Chemical Society, Perkin Transaction I, 1988, pp. 1407-1415; Journal of Chemical Society, Perkin Transaction I, 1989, pp. 376-377 and Journal of Chemical Society, Perkin Transaction I, 1990, pp. 673-679), and carbazole synthesis using vinyl indole (Pindur et al., Helvetica Chimica Acta, 1988, vol. 71, pp. 1060-1064; and Journal of Organic Chemistry, 1990, vol. 55, pp. 5368-5374).

Although the above-mentioned methods are excellent as a condensed ring-forming reaction in that the condensed ring can be formed by a single stage, the method of Magnus et al. is only used in the intramolecular Diels-Alder reaction, while the method of using pyranoindol-3-one or vinyl indole requires multi-stages and/or special steps for the preparation of the starting materials and further requires expensive starting materials and reaction agents. From an industrial point of view these methods do not take full advantage of the single stage cyclization reaction. Further, each of the processes is applied only to the carbazole derivatives, and can not be said to be a general method for synthesizing condensed ring compounds.

Thus, it is an object of the present invention to provide a novel intermediate product which can be induced to various condensed ring compounds.

Another object of the present invention is to provide a process for producing such intermediate products in short steps and at a high yield taking advantage of the feature of the cycloaddition reaction, using easily available starting materials and without using expensive reaction reagents.

The present invention concerns:
(1) 1-amino-2-cyclohexene derivatives represented by the following general formula (I): 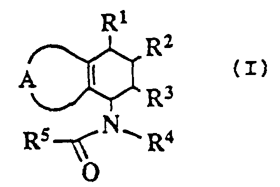 (where A represents a bivalent organic group which may contain 1 to 3 oxygen atoms, nitrogen atoms and/or sulfur atoms, in which A may form a 5-membered ring, 6-membered ring, 7-membered ring or 8-membered ring together with two carbon atoms to be bonded, and the ring may form a condensed ring with one or plurality of other rings; R¹ represents a hydrogen atom, an alkyl group, an alkenyl group, an aryl group or an aralkyl group, R² represents a hydrogen atom, an alkyl group, an alkenyl group, an aryl group, an aralkyl group, a cyano group or a group represented by the formula: -COR²¹, R²¹ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, R³ represents a cyano group, a nitro group or a group represented by the formula: -COR³¹, R³¹ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, R⁴ represents an alkyl group, an alkenyl group, an aryl group or an aralkyl group, R⁵ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, in which R² and R³¹ may join together to form a bivalent organic group possibly containing an oxygen atom or a nitrogen atom) [hereinafter sometimes referred to as a 1-amino-2-cyclohexene derivative (I)], (2) a process for producing an 1-amino-2-cyclohexene derivative (I) which comprises condensating an aldehyde represented by the general formula (II): 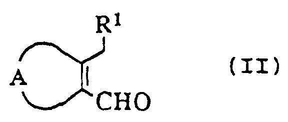 (where A and R¹ are as defined above) [hereinafter sometimes referred to as an aldehyde (II)] with a primary amine represented by the following general formula (III):

H₂NR⁴ (III)

(where R⁴ is as defined above) [hereinafter sometimes referred to as an amine (III)] to obtain an imine represented by the following general formula (IV): 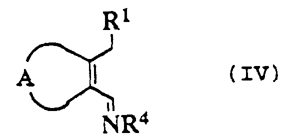 (where A, R¹ and R⁴ are as defined above) [hereinafter sometimes referred to as an imine (IV)], and reacting the imine (IV) in the presence of a basic substance with a carbonylating agent represented by the following general formula (V): 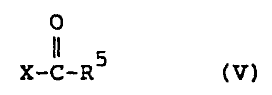 (where R⁵ is as defined above and X represents a leaving group) [hereinafter sometimes referred to as a carbonylating agent (V)] and an ethylene derivative represented by the following general formula (VI):

R²-CH=CH-R³ (VI)

(where R² and R³ are as defined above) [hereinafter sometimes referred to as a dienophilic agent (VI)], and (3) a process for producing a 1,3-cyclohexadiene derivative represented by the following general formula (IX): 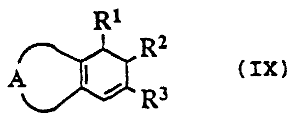 (where A, R¹, R² and R³ are as defined above) [hereinafter sometimes referred to as a 1,3-cyclohexadiene derivative (IX)], which comprises subjecting the 1-amino-2-cyclohexene derivative (I) to an elimination reaction by a basic substance.

In the general formula (I), the general formula (II), the general formula (IV) and the general formula (IX) described above, as concrete examples of a ring formed by A together with two carbon atoms to be bonded, there can be mentioned, for example, 5-membered ring such as a cyclopentene ring, cyclopentadiene ring, dihydrofuran ring, furan ring, pyrrole ring, pyrroline ring, dehydrodioxolane ring, pyrazole ring, pyrazoline ring, imidazole ring, oxazole ring, isoxazole ring, thiazole ring, oxadiazole ring and a triazole ring; a 6-membered ring such as a benzene ring, cyclohexadiene ring, cyclohexene ring, pyran ring, dihydropyran ring, pyridine ring, dihydropyridine ring, tetrahydropyridine ring, dehydrodioxan ring, dehydromorpholine ring, pyridazine ring, dihydropyridazine ring, tetrahydropyridazine ring, pyrimidine ring, dihydropyrimidine ring, tetrahydropyrimidine ring, pyrazine ring and a dihydropyrazine ring; a 7-membered ring such as a cycloheptatriene ring, cycloheptadiene ring, cycloheptene ring, as well as aza substitutent, oxa substitutent or thia substituent thereof, and thiazepin ring; and a 8-membered ring such as a cyclooctatetraene ring, cyclooctatriene ring, cyclooctadiene ring, cyclooctene ring as well as an aza substituent, oxa substituent or thia substituent thereof. Further, as concrete examples of the condensed ring in a case where the ring formed by A together with two carbon atoms to be bonded forms a condensed ring with one or plurality of other rings, there can be mentioned, for example, a benzofuran ring, isobenzofuran ring, chromene ring, indolizine ring, isoindole ring, indole ring, quinolizine ring, indazole ring, isoquinoline ring, quinoline ring, phthalazine ring, naphthyridine ring, quinoxaline ring, quinazoline ring, benzothiophene ring and hydrogenated forms thereof. Any of the rings may have a substituent.

In the general formula (I), the general formula (II), the general formula (III), the general formula (IV), the general formula (V), the general formula (VI) and the general formula (IX), as the alkyl group which may be represented by R¹, R², R²¹, R³¹, R⁴ and R⁵, respectively, there can be mentioned, for example,a linear or branched alkyl group of 1 to 8 carbon atoms such as a methyl group, ethyl group, propyl group, isopropyl group, butyl group, isobutyl group, sec-butyl group, tert-butyl group, pentyl group, isopentyl group, neopentyl group, hexyl group, heptyl group and an octyl group; and a cycloalkyl group such as cyclopropyl group, cyclobutyl group, cyclopentyl group and a cyclohexyl group. As the alkenyl group which may be represented by R¹, R², R²¹, R³¹, R⁴ and R⁵, respectively, there can be mentioned, for example, a vinyl group, allyl group, methallyl group, butenyl group, prenyl group and an octenyl group.

In the general formula (I), the general formula (II), the general formula (III), the general formula (IV), the general formula (V), the general formula (Vl) and the general formula (IX), as the aryl group which may be represented by R¹, R², R²¹, R³¹, R⁴ and R⁵, there can be mentioned, for example, a phenyl group or naphthyl group which may have a substituent, for example, a phenyl group, naphthyl group, fluorophenyl group, chlorophenyl group, bromophenyl group, methoxyphenyl group, nitrophenyl group, tolyl group, xylyl group and an isopropylphenyl group. As the aralkyl group which may be represented by R¹, R², R²¹, R³¹, R⁴ and R⁵ respectively, there can be mentioned, for example, benzyl group which may have a substituent such as benzyl group, methoxybenzyl group, dimethoxybenzyl group, nitrobenzyl group, chlorobenzyl group and a bromobenzyl group.

In the general formula (I), the general formula (V), the general formula (VI) and the general formula (IX), as alkoxyl group which may be represented by R²¹, R³¹ and R⁵, respectively, there can be mentioned, for example, linear or branched alkoxyl group of 1 to 8 carbon atoms such as a methoxy group, ethoxy group, propoxy group, isopropoxy group, butoxy group, hexyloxy group and an octyloxygroup; and a cycloalkyloxy group such as a cyclopentyloxy group and cyclohexyloxy group. As the alkenyloxyl group which may be represented by R²¹, R³¹ and R⁵, respectively, there can be mentioned, for example, an alkenyloxyl group of 1 to 8 carbon atoms such as an allyloxy group, methallyloxy group, prenyloxy group and an octenyloxy group.

Further, in the general formula (I), the general formula (V), the general formula (VI) and the general formula (IX), as the aryloxy group which may be represented by R²¹, R³¹ and R⁵, respectively, there can be mentioned, for example,a phenoxy group which may have a substituent such as a phenoxy group, methylphenoxy group, methoxyphenoxy group, chlorophenoxy group, bromophenoxy group and a nitrophenoxy group. As the aralkyloxyl group which may be represented by R²¹, R³¹ and R⁵, respectively, there can be mentioned, for example, benzyloxy group which may have a substituent such as a benzyloxy group, chlorobenzyloxy group, bromobenzyloxy group, methoxybenzyloxy group, methylbenzyloxy group and nitrobenzyloxy group. As the amino group having the substituent which may be represented by R²¹, R³¹ and R⁵, respectively, there can be mentioned, for example, a secondary amino group substituted with an aralkyl group, alkylene group, aryl group and/or aralkyl group such as a dimethylamino group, diethylamino group, N-phenylmethylamino group, N-benzylmethylamino group and a 1-pyrrolidyl group.

As the leaving group represented by X in the general formula (V), there can be mentioned, for example, a halogen atom such as chlorine atom and bromine atom; and an acyloxy group such as acetoxy group, propionyloxy group, butylyloxy group and a valeryloxy group.

As a typical example of the 1-aminno-2-cyclohexene derivative (I), there can be mentioned a tetrahydrobenzothiophene derivative or a tetrahydrobenzofuran derivative represented by the following general formula (I-1): 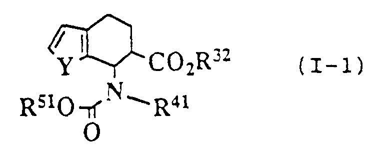 (where each of R³², R⁴¹ and R⁵¹ represents an alkyl group, an aryl group or an aralkyl group and Y represents a sulfur atom or an oxygen atom), and a tetrahydrocarbazole derivative represented by the following general formula (I-2): 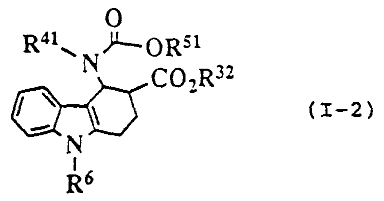 (where R³², R⁴¹ and R⁵¹ are as defined above, and R⁶ represents an alkyl group, an aralkyl group, an acyl group, an alkoxycarbonyl group, an alkanesulfonyl group or an arenesulfonyl group).

As the concrete example of the aldehyde (II), there can be mentioned, for example, a 3-methylthiophene-2-aldehyde or a 3-methylfuran-2-aldehyde represented by the following formula (VII): 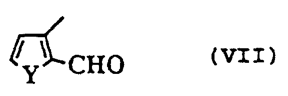 (where Y is as defined above), or a 2-methylindole-3-aldehyde represented by the following formula (VIII):

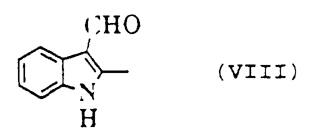

As a concrete example of the 1,3-cyclohexadiene derivative (IX), a dihydrobenzothiophene carboxylic acid derivative or a dihydrobenzofuran carboxylic acid derivative represented by the following general formula (IX-11): 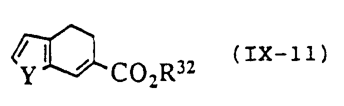 (where R³² and Y are as defined above) [hereinafter sometimes referred to as a compound (IX-11)], or a dihydrocarbazol carboxylic acid derivative represented by the following general formula (IX-21): 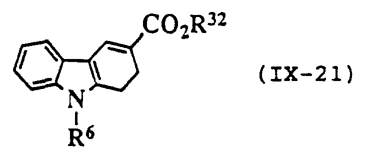 (where R³² and R⁶ are as defined above) [hereinafter sometimes referred to as a compound (IX-21)] may be mentioned.

The compound (IX-11) and the compound (IX-21) can be transformed by subjecting it, if necessary, to a hydrolyzing reaction into a dihydrobenzothiophene carboxylic acid derivative or a dihydrobenzofuran carboxylic acid derivative represented by the following general formula (IX-1): 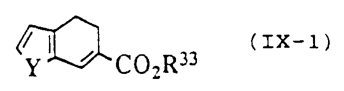 (where Y is as defined above and R³³ represents a hydrogen atom, an alkyl group, an aryl group or an aralkyl group), or a dihydrocarbazol carboxylic acid derivative represented by the following general formula (IX-2) : 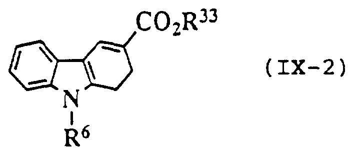 (where R³³ and R⁶ are as defined above), respectively.

In the general formula (I-1), the general formula (I-2), the general formula (IX-1), the general formula (IX-11), the general formula (IX-2) and the general formula (IX-21), as the alkyl group, the aryl group and the aralkyl group represented by R³², R³³, R⁴¹ and R⁵¹, respectively, there can be mentioned the same groups as the alkyl group, the aryl group and the aralkyl group which may be represented by R¹, R², R²¹, R³¹, R⁴ and R⁵.

In the general formula (I-2), the general formula (IX-2) and the general formula (IX-21), as the group which may be represented by R⁶, there can be mentioned, for example, a group used generally as a protection group for the nitrogen atom of an indole. More specifically, as the alkyl group, there can be mentioned, an alkyl group which may be represented by R¹, R², R²¹, R³¹, R⁴ and R⁵. As the aralkyl group, there can be mentioned, for example, an aralkyl group which may be represented by R¹, R², R²¹, R³¹, R⁴ and R⁵. As the acyl group, there can be mentioned, for example, an alkanoyl group which may have a substituent such as a formyl group, acetyl group, propionyl group, butyryl group, isobutyryl group, valeryl group, isovaleryl group, pivaloyl group, chloroacetyl group and a trifluoroacetyl group; and a benzoyl group or a naphthoyl group which may have a substituent such as a benzoyl group, methoxybenzoyl group, chlorobenzoyl group and naphthcyl group. As the alkoxycarbonyl group, there can be mentioned a lower alkoxy, for example, a lower alkoxycarbonyl group such as a methoxycarbonyl group, ethoxycarbonyl group, propoxycarbonyl group, isopropoxycarbonyl group, butoxycarbonyl group and a tert-butoxycarbonyl group; an aryloxycarbonyl group which may have a substituent such as a phenoxycarbonyl group and a nitrophenoxycarbonyl group; and an aralkyloxycarbonyl group which may have a substituent such as a benzyloxycarbonyl group and a methoxybenzyloxycarbonyl group. As the alkane sulfonyl group, there can be mentioned, for example, a methane sulfonyl group and an ethane sulfonyl group. As the arene sulfonyl group, there can be mentioned, for example, a benzene sulfonyl group, toluene sulfonyl group and a bromobenzene sulfonyl group.

Conversion from the aldehyde(II) to the imine (IV) is conducted by condensating the aldehyde (II) with the amine (III). The conversion can be conducted in the same manner as the method used generally for obtaining an imine from an aldehyde and a primary amine. For example, the conversion can be conducted by mixing the aldehyde (II) and the amine (III) in the presence or absence of a solvent giving no undesired effect on the reaction, for example, an aliphatic hydrocarbon solvent such as pentane, hexane, heptane and ligroin; an aromatic hydrocarbon solvent such as benzene, toluene, xylene and chlorobenzene; an ether solvent such as diethyl ether, tetrahydrofuran and dioxane; an alcohol solvent such as methanol and ethanol; an ester solvent such as methyl acetate, ethyl acetate and butyl acetate; or a solvent comprising a mixture of them, and reacting in the presence or absence of a dehydrating agent, for example, silica gel, molecular sieves, alumina, sodium sulfate, magnesium sulfate and copper sulfate.

Further,the reaction may also be conducted in an azeotropic solvent with water while removing water by azeotropic dehydration.

Isolation and purification from the reaction mixture of the thus obtained imine (IV) is conducted in the same manner as the method usually used in isolation and purification of organic compounds. For instance, the imine (IV) can be obtained by separating insoluble matters contained in the reaction mixture by filtration, condensating the liquid filtrate and then recrystallizing residues and then purifying, for example, by chromatography. Further, crude products can be used without purification to the succeeding reaction as they are. When the resultant imine (IV) is deposited from the reaction mixture, it is recovered by filtration and purified if necessary by recrystallization and then can be used to the succeeding reaction.

Conversion from the imine (IV) to the 1-amino-2-cyclohexene derivative (I) is conducted in the presence of a basic substance by reacting the imine (IV) with the carbonylating agent (V) and the dienophilic agent (VI). As the carbonylating agent used herein, there can be mentioned, for example, a carboxylic acid anhydride such as acetic anhydride, propionic anhydride, butyric anhydride, valeric anhidride and trifluoro acetic anhydride; a carboxylic acid halide such as acetyl chloride, propionyl chloride, butyryl chloride, isobutyryl chloride, valeryl chloride, isovaleryl chloride, pivaroyl chloride and benzoyl chloride; a chloroformate ester such as methyl chloroformate, ethyl chloroformate, propyl chloroformate, isopropyl chloroformate, butyl chloroformate, allyl chloroformate, phenyl chloroformate, nitrophenyl chloroformate and benzyl chloroformate; a carbamic acid halide such as N,N-dimethyl carbamic acid chloride. Among them, chloroformate ester is suitably used. The amount of the carbonylating agent (V), while different depending on the type, ranges usually from 0.5 to 20 mol, preferably, from 1.1 to 10 mol based on one mol of the imine (IV).

As the dienophilic agent (VI) used for such reaction, there can be mentioned, for example, an acrylate which may have a substituent, an acrylamide which may have a substituent, an acrylonitrile which may have a substituent, a propenal which may have a substituent, a vinyl ketone which may have a substituent, maleic acid ester, maleic anhydride, maleimide, fumaric acid ester, fumaronitrile and nitroethylene which may have a substituent. More specifically, there can be mentioned, for example, an alkyl acrylate such as methyl acrylate, ethyl acrylate, propyl acrylate, isopropyl acrylate, butyl acrylate, isobutyl acrylate, sec-butyl acrylate, tert-butyl acrylate, pentyl acrylate, hexyl acrylate, cyclopentyl acrylate and cyclohexyl acrylate; an aryl acrylate such as phenyl acrylate, naphthyl acrylate, chlorophenyl acrylate, bromophenyl acrylate, methoxyphenyl acrylate, nitrophenyl acrylate, tolyl acrylate, xylyl acrylate and isopropylphenyl acrylate; an aralkyl acrylate such as benzyl acrylate, methoxybenzyl acrylate, dimethoxybenzyl acrylate, nitrobenzyl acrylate, chlorobenzyl acrylate and bromobenzyl acrylate; a substituted acrylate such as methyl crotonate and methyl succinate; an N-substituted acrylamide such as N,N-dimethylacrylamide;a substituted acrylamide such as N,N-dimethylcrotonamide and N,N-dimethylcinnamamide; acrylonitrile; an aromatic-substituted acrylonitrile such as crotononitrile and cinnamonitrile; acrolein; a substituted propenal such as crotonaldehyde and cinnamaldehyde; vinyl ketones such as methyl vinyl ketone, ethyl vinyl ketone, phenyl vinyl ketone, styryl methyl ketone, 3-penten-2-one and 1-penten-3-one; a maleate such as dimethyl maleate; maleic anhydride; a fumarate such as dimethyl fumarate; a maleimide such as N-phenyl maleimide; a fumaronitrile and vinyl nitro compound such as nitroethylene. The amount of the dienophilic agent (VI) used ranges usually from 0.5 to 50 mol, preferably, from 1.1 to 10 mol based on 1 mol of the imide (IV).

As the basic substance used for such a reaction, there can be mentioned, for example, a tertiary amine such as trimethyl amine, triethyl amine, tributyl amine, trihexyl amine, trioctyl amine, diisopropyl ethyl amine, dimethyl aniline, diethyl aniline and N-methylmorpholine; an alkali metal carbonate such as lithium carbonate, sodium carbonate and potassium carbonate; an alkali metal hydroxide such as lithium hydroxide, sodium hydroxide and potassium hydroxide; an alkaline earth metal hydroxide such as magnesium hydroxide and calcium hydroxide; and a metal hydride such as a lithium hydride and sodium hydride. Among them, a tertiary amine which is sterically bulky, exhibits relatively high basicity and is soluble to a reaction system, such as diisopropyl ethyl amine, is preferred. The amount of the basic substance used ranges usually from 0.5 to 50 mol, preferably, from 1.1 to 10 mol based on one mol of the imine (IV) and it is preferred to add more than 1 mol based on 1 mol of the carbonylating agent (V) used.

The conversion reaction from the imine (IV) to 1-amino-2-cyclohexene derivative (I) can be conducted with or without solvent. When the reaction is conducted in a solvent, there is no particular restriction on the solvent to be used so long as it does not give any undesired effect on the reaction and there can be mentioned, for example, an aromatic hydrocarbon solvent such as benzene, toluene, xylene, chlorobenzene, trimethylbenzene and cumene. The amount of the solvent used usually ranges from 3 to 200 times by weight based on the imine (IV).

The reaction temperature may vary depending on the kind of the solvent, the carbonylating agent (V) and the dienophilic agent (VI) used and it is preferably within a range usually from 40°C to the refluxing temperature for the reaction system. The reaction time, which may vary depending on the reaction temperature, usually ranges from 30 minutes to 24 hours. By properly controlling the reaction temperature, the reaction time can be controlled.

The conversion reaction described above is practiced, for example, as shown below. The dienophilic agent (VI) and the basic substance are added to the solution of the imine (IV), and the carbonylating agent (V) is dropped to the resultant mixture within a temperature range from under ice cooling to a refluxing temperature. Thereafter, the mixture is heated to a desired temperature till the imine (IV) disappears.

Isolation and purification of the thus obtained 1-amine-2-cyclohexene derivative (I) from the mixture is conducted in the same manner as the method used usually for isolation and purification of organic compounds. For instance, after cooling the reaction mixture to a room temperature, it is washed with an aqueous solution of sodium hydrogen carbonate and saline water, dried on sodium sulfate and then the solvent is distilled off to obtain a crude product.

Conversion from the 1-amino-2-cyclohexene derivative (I) to 1,3-cyclohexadiene derivative (IX) is conducted in the presence of a basic substance by subjecting 1-amino-2-cyclohexene derivative (I) to an elimination reaction.

The basic substance used for the elimination reaction has no particular restriction so long as the substance does not cause side reaction and has such a sufficient basicity to be capable of forming the 1,3-cyclohexadiene derivative (IX) by an elimination reaction. There can be mentioned, for example, a metal alkoxide such as lithium methoxide, sodium methoxide, potassium methoxide, sodium ethoxide, potassium ethoxide, potassium tert-butoxide and sodium tert-amyloxide; a metal amide such as lithium amide, sodium amide, potassium amide, lithium diisopropyl amide, lithium cyclohexyl isopropyl amide, lithium hexamethyl disilazide, sodium hexamethyl disilazide, potassium hexamethyl disilazide and lithium tetramethyl piperizide; an amine such as diazabicyclo[2.2.2]octane (DABCO) and diazabicyclo[5.4.0]undec-7-ene (DBU); and a quaternary ammonium hydroxide such as trimethyl benzyl ammonium hydroxide, and tributyl ammonium hydroxide. In the case of the quarternary ammonium hydroxide, it can be formed in the system from a corresponding halide and an alkali metal hydroxide in the system. The amount of the basic substance used ranges usually from 0.8 to 20 mol, preferably, from 0.95 to 10 mol based on one mol of the 1-amino-2-cyclohexene derivative (I).

The elimination reaction is conducted usually in a solvent and the solvent used varies depending on the kind of the basic substance used and there can be mentioned, for example, an alcohol solvent such as methanol, ethanol, propanol, isopropyl alcohol and tert-butyl alcohol; an ether solvent such as diethyl ether, tetrahydrofuran, dioxane and 1,2-dimethoxy ethane; a polar aprotic solvent such as N,N-dimethylformamide and dimethyl sulfoxide; a hydrocarbon solvent such as hexane, heptane, cyclohexane, petroleum ether and ligroin; an aromatic hydrocarbon solvent such as benzene, toluene and xylene; water; or a solvent comprising a mixture thereof. The amount of the solvent used usually ranges from 3 to 200 times by weight based on the 1-amino-2-cyclohexene derivative (I).

The reaction temperature for the elimination reaction, while different depending on the basic substance and the solvent used, usually ranges from 0°C to 150°C. Further, the reaction time varies depending on the reaction temperature and it usually ranges from 30 minutes to 24 hours.

Isolation and separation of the thus obtained 1,3-cyclohexadiene derivative (IX) from the reaction mixture is usually conducted in the same manner as the method used usually in isolation and purification of organic compounds. For instance, the 1,3-cyclohexadiene derivative (IX) can be obtained by adding the reaction mixture to ice water, separating an organic layer, then extracting an aqueous layer with an organic solvent such as ethyl acetate, diethyl ether, dichloromethane and toluene, collecting the organic layer and washing with an aqueous sodium chloride solution, drying over sodium sulfate or magnesium sulfate and then concentrating to obtain a crude product, which is purified by recrystallization, chromatography or the like. Further, it is possible to use the crude products with no purification as they are to the succeeding reaction or use the reaction mixture without isolation or purification as it is to the succeeding reaction.

The 1,3-cyclohexadiene derivative (IX) in which R³ represents a group represented by the formula -COR³¹, and R³¹ represents an alkoxyl group, an alkenyloxyl group, an aryloxyl group or an aralkyloxyl group, can be converted by hydrolyzing reaction into a 1,3-cyclohexadiene carboxylic acid derivative represented by the following general formula (IX-3): 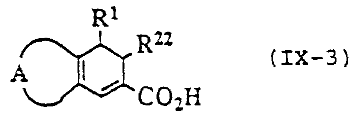 (where A and R¹ are as defined above, R²² represents R² or carboxyl group, and R² is as defined above) [hereinafter sometimes referred to as a carboxylic acid (IX-3)]. The carboxylic acid (IX-3) is a compound included in the 1,3-cyclohexadiene derivative (IX).

The hydrolyzing reaction can be conducted in accordance with a method used generally upon conversion of an ester to a corresponding carboxylic acid. For instance, it can be conducted by adding an aqueous solution containing a sufficient amount of an alkali metal hydroxide for hydrolysis to the 1,3-cyclohexadiene derivative (IX) in which the group represented by R³ is an alkoxy carbonyl group, an alkenyloxy carbonyl group, an aryloxy carbonyl group and an aralkyloxy carbonyl group, or a solution thereof, and stirring at a temperature within a range from 0°C to 100°C until the 1,3-cyclohexadiene derivative (IX) in which the group represented by R³ is an alkoxy carbonyl group, an alkenyloxy carbonyl group, an aryloxy carbonyl group, an aralkyloxy carbonyl group disappears completely.

Isolation and purification of the thus obtained carboxylic acid (IX-3) from the reaction mixture can be conducted in the same manner as the method used generally in isolation and purification of organic compounds. For instance, a crude product is obtained by distilling off low boiling ingredients of the reaction mixture, adding water as necessary to the resultant residue, extracting the same with an organic solvent such as ethyl acetate, diethyl ether, dichloromethane and toluene, rendering the aqueous layer acidic, for example, with hydrochloric acid, then extracting with an organic solvent such as ethyl acetate, diethyl ether and dichloromethane, washing the liquid extract with an aqueous sodium chloride solution, then drying by using sodium sulfate or the like and then distilling off the solvent. Then, the crude product is purified, for example, by chromatography or recrystallization to obtain the carboxylic acid (IX-3).

The thus obtained carboxylic acid (IX-3) can be converted into the 1,3-cyclohexadiene derivative (IX) by esterification in accordance with a customary method.

Among the 1,3-cyclohexadiene derivatives (IX), the compounds represented by the following formula (IX-12): 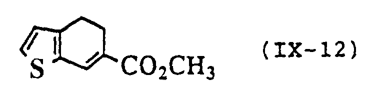 can be converted into 2-(1-imidazolylmethyl)-4,5-dihydrothianaphthene-6-carboxylic acid which has an inhibitory action on thromboxane synthetase and is useful as a pharmaceutical medicine in accordance with the method as described, for example, in Journal of Medicinal Chemistry, 1989, vol. 32, pp. 1265-1272. Further, the compound represented by the following general formula (IX-13): 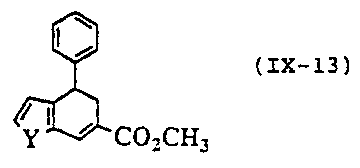 (where Y is as defined above), can be converted by dehydrogenation reaction, hydrolysis and amidation by sec-butylamine and N-methylation into N-sec-butyl-N-methyl-5-phenylbenzo[b]furan-6-carboxamide or N-sec-butyl-N-methyl-5-phenylhenzo[b]thiophene-6-carboxamide having tranquilizer, anti-aginal and immunomodulator activity. Further, the compound represented by the following formula (IX-14): 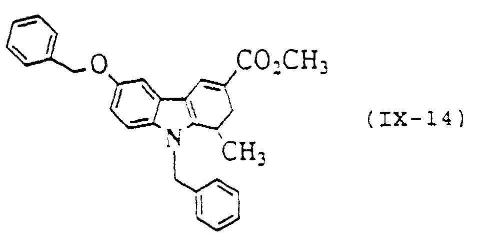 can be converted by conjugate addition of cyanide ion, hydrolysis, imidation, debenzylation and dehydrogenating reaction into N-(N-dimethylaminoethyl)-8-hydroxy-1-methyl-9H-carbazol-3,4-dicarboximide having anti-tumor activity.

The aldehyde (II) can be obtained, for example, by formylating the compound represented by the following general formula (X): 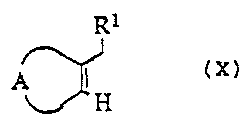 (where A and R¹ are as defined above). For instance, 3-methylthiophene-2-aldehyde can be obtained from 3-methylthiophene, 3-methylfuran-2-aldehyde can be obtained from 3-methylfuran and 2-methylindole-3-aldehyde can be obtained from 2-methylindole by formylation using a Vilsmeier reagent (N,N-dimethylformamide-phosphoroxy chloride), respectively. The aldehydes are available as commercial products.

### EXAMPLES

The present invention will be explained specifically with reference to examples but the invention is not limited only to the examples.

### Example 1

To 3-methylthiophene-2-aldehyde (103 g, 0.816 mol) was mixed with hexane (100 ml), aniline (74.4 ml, 0.816 mol) was added and stirred at room temperature for 7 hours. After separating deposited crystalline solids by filtration, and washing with hexane, 139.5 g (yield: 85%) of 3-methylthiophene-2-aldehyde phenylimine was obtained. After concentrating the liquid filtrate and stirring at room temperature for further 12 hours, 7.30 g (yield: 4%) of 3-methylthiophene-2-aldehyde phenylimine was obtained by post treatment in the same manner.
¹H-NMR spectra (chemical shift, ppm: in CDCl₃);
2.47 (3H, s), 6.92 (1H, d, J=4.9Hz), 7.15-7.30 (3H), 7.30-7.45 (3H), 8.61 (1H).

3-methylthiophene-2-aldehyde phenylimine (50 g, 0.248 mol), methyl acrylate (224 ml, 2.48 mol) and diisopropyl ethylamine (173 ml, 0.993 mol) were mixed in xylene (1000 ml) and stirred under ice cooling. Methyl chloroformate (76.8 ml, 0.993 mol) was dropped to the mixture. Thereafter, the reaction mixture was heated under reflux for 3 hours and then allowed to cool to room temperature. The reaction mixture was washed with an aqueous solution of sodium hydrogencarbonate and an aqueous solution of sodium chloride and dried over sodium sulfate. By distilling off the solvent, 83 g of crude product of N-methoxycarbonyl-N-phenyl-7-amino-4,5,6,7-tetrahydrobenzo[b]thiophene-6-carboxylic acid methyl ester was obtained.

The thus obtained crude N-methoxycarbonyl-N-phenyl-7-amino-4,5,6,7-tetrahydrobenzo[b]thiophene-6-carboxylic acid methyl ester (83 g) was dissolved in methanol (830 ml), and sodium methoxide (95.8 g in 28% methanol solution) was added and stirred at 60°C for 2 hours. The mixture was then allowed to cool to room temperature. Water (500 ml) and potassium hydroxide (27.9 g) were added to the reaction mixture and stirred at 60°C for one hour. After distilling off methanol, water was added to the residue and extracted with toluene. The resultant aqueous layer was rendered acidic with diluted hydrochloric acid and extracted with ethyl acetate. The liquid extract was washed with an aqueous solution of sodium chloride and then dried over sodium sulfate. The solvent was distilled off and the resultant residue was purified by recrystallization (in methyl acetate /hexane) to obtain 30.9 g (yield: 72.3% from phenylimine) of 4,5-dihydrobenzo[b]thiophene-6-carboxylic acid as a pale yellow crystalline powder. By concentrating and then recrystallizing the filtrate, 4.3 g (yield: 10.1%) of crystals was obtained.
Melting point: 151.5-153°C
¹H-NMR spectra (chemical shift, ppm: in CDCl₃):
2.70 (2H, m), 2.88 (2H, m), 6.93 (1H, d, J=4.9Hz), 7.37 (1H, d, J=4.9Hz), 7.66 (1H, s).

### Condition for high speed liquid chromatographic analysis:

Column: Hiber LiCrospher 100 RP-18 (5 µm) 250 mm x 4 mm φ (manufactured by Cica-MERCK)
Eluent: methanol / water (volume ratio 1:1) 0.9 ml/min
Column temperature: 45°C
Detector: UV absorption detector
Wavelength: 254 nm
Retention time: 6.1 min
Purity (surface area percentage): 99.6%

### Example 2

3-methylthiophene-2-aldehyde phenylimine (1.92 kg, 9.48 mol), diisopropyl ethyl amine (4.96 kg, 38.34 mol), methyl acrylate (4.13 kg, 47.94 mol) and methyl chloroformate (3.63 kg, 38.4 mol) were mixed in xylene (30 liter) at room temperature and stirred at 80-100°C under reflux for 3 hours. After allowed to cool to room temperature, sodium hydrogencarbonate (10% aqueous solution,10 liter) and water (7 liter) were added, and after washing the organic layer with sodium chloride (20% aqueous solution), N-methoxycarbonyl-N-phenyl-7-amino-4,5,6,7-tetrahydrobenzo[b]thiophene-6-carboxylic acid methyl ester was obtained by filtration as a crystalline solid (660 g, yield: 20% from imine).
¹H-NMR spectra (chemical shift, ppm: in CDCl₃):
0.95-1.23, 1.65-1.85 (joined 1H, m), 1.85-2.25 (1H, m), 2.25-2.75 (2H, m), 2.75-3.20 (1H, m), 3.50-4.00 (1H, br), 3.71 (3H, s), 3.82 (3H, s), 6.85-7.40 (joined 1H, m), 6.60-6.95 (2H, m), 6.95-7.60 (4H, m).

### Example 3

N-methoxycarbonyl-N-phenyl-7-amino-4,5,6,7-tetrahydrobenzo[b]thiophene-6-carboxylic acid methyl ester (100 mg, 0.290 mmol) was dissolved in methanol (3 ml), to which sodium methoxide (28% methanol solution, 1 ml) was added and stirred at room temperature overnight. The reaction mixture was poured into iced water and extracted with diethyl ether. After washing the liquid extract with an aqueous solution of sodium chloride and drying over sodium sulfate, the solvent was distilled off. The residue was purified by silica gel column chromatography to obtain 46.7 mg (yield 48%) of 4,5-dihydrobenzo[b]thiophene-6-carboxylic acid methyl ester as a white crystalline solid.
¹H-NMR spectra (chemical shift, ppm: in CDCl₃): 2.68 (2H, m), 2.84 (2H, m), 3.79 (3H, s), 6.90 (1H, d, J=4.9Hz), 7.31 (1H, d, J=4.9Hz), 7.53 (1H, s).

### Example 4

4,5-dihydrobenzo[b]thiophene-6-carboxylic acid methyl ester (100 mg, 0.515 mmol) was dissolved in methanol (5 ml), to which potassium hydroxide (1N aqueous solution, 2 ml) was added and stirred under heating under reflux for 3 hours. After allowing the reaction mixture to cool, the methanol was distilled off. The resultant mixture was diluted with water and extracted with toluene. The aqueous layer was rendered acidic by using 1N hydrochloric acid and extracted with ethyl acetate. After washing the liquid extract with an aqueous solution of sodium chloride and drying it over sodium sulfate, the solvent was distilled off. 76 mg (yield: 82%) of 4,5-dihydro[b]thiophene-6-carboxylic acid showing the same physical property values as those obtained in Example 1 were obtained as a crystalline solid by recrystallizing the residue (in methyl acetate / hexane).

### Examples 5-9

Table 1 shows results of analysis by high speed chromatography when the reaction was conducted in the same manner as in Example 2 using various basic substances. In the table, the imine represents 3-methylthiophen-2-aldehyde phenylimine, the aldehyde represents 3-methylthiophene-2-aldehyde, the carbamate represents methyl N-phenyl carbamate, the tetrahydrobenzothiopene represents N-methoxycarbonyl-N-phenyl-4,5,6,7-tetrahydrobenzo[b]thiophene-6-carboxylic acid methyl ester.

| Example No. | Basic substance | | Imine-based equivalent of methyl chloro formate | Reaction time (Hr) | HPLC area ratio (%) | | | |
|---|---|---|---|---|---|---|---|---|
| | Compound | Imine-based equivalent | | | Imine | Aldehyde | Carbamate | Tetrahydrobenzothiophene |
| 5 | NaOH | 4 | 2 | 0.5 | 0 | 31 | 14 | 54 |
| 6 | Na₂CO₃ | 4 | 2 | 5 | 0 | 16 | 6 | 78 |
| 7 | K₂CO₃ | 5 | 4 | 0.5 | 0 | 41 | 15 | 44 |
| 8 | K₂CO₃ | 10 | 2 | 0.2 | 0 | 60 | 20 | 20 |
| 9 | LiH | 4 | 2 | 1.5 | 0 | 27 | 11 | 62 |

### Condition for high speed liquid chromatographic analysis:

Column: Hiber LiCrosorb Si-60 (5 µm)
   250 mm x 4 mm φ (manufactured by Cica-MERCK)
Column temperature: 32°C
Eluent: hexane / tetrahydrofuran (volume ratio 10:1) 1 ml/min
Detector: UV absorption detector
Wavelength: 254 nm

### Example 10

To 3-methylthiophene-2-aldehyde phenylimine (100 mg, 0.497 mmol) and triethylamine (0.21 ml, 1.49 mmol) were mixed in xylene, acetyl chloride (0.14 ml, 1.99 mmol) was added under ice cooling. Methyl acrylate (0.22 ml, 2.48 mmol) was added to the resultant mixture and stirred and heated under reflux for 7 hours. After allowing the reaction mixture to cool, water was added and the mixture was extracted with ethyl acetate. After washing the liquid extract with an aqueous solution of sodium chloride and drying over sodium sulfate, the solvent was distilled off to obtain 300 mg of crude products. They were purified by silica gel column chromatography to obtain 114 mg (yield: 83%) of N-acetyl-N-phenyl-7-amino-4,5,6,7-tetrahydrobenzo[b] thiophene-6-carboxylic acid methyl ester.
¹H-NMR spectra (chemical shift, ppm: in CDCl₃):
1.00-1.50 (1H, m), 1.73, 1.86 (joined 3H, s x 2), 1.85-2.20 (1H, m), 2.30-2.85 (2.5H), 2.95-3.20 (0.5H, m), 3.74, 3.82 (joined 3H, s x 2), 6.63-6.85 (2H), 7.15-7.70 (5H).

### Example 11

N-acetyl-N-phenyl-7-amino-4,5,6,7-benzo[b]-thiophene-6-carboxylic acid methyl ester (134 mg, 0.407 mmol) was dissolved in methanol (5 ml), to which sodium methoxide (28% methanol solution; 157 mg, 0.813 mmol) was added and stirred at room temperature. Then, sodium methoxide (28% methanol solution, 0.16 ml) was added after 4 hr, 10 hr, 28.5 hr respectively and the mixture was stirred for 35.5 hours at room temperature. After heating the reaction mixture for 45 minutes under reflux, it was allowed to cool to room temperature. The reaction mixture was poured into iced water and extracted with diethyl ether. After washing the liquid extract with an aqueous solution of sodium chloride and drying it over sodium sulfate, the solvent was distilled off to obtain 250 mg of crude products. They were purified by silica gel column chromatography to obtain 66 mg (yield: 84%) of 4,5-dihydrobenzo[b]thiophene-6-carboxylic acid methyl ester showing the same physical property values as those obtained in Example 3.

### Example 12

2-methylindole-3-carboxaldehyde (10 g, 0.06 mol) was mixed with hexane (40 ml), to which aniline (9.6 ml, 0.11 mol) was added and stirred at 70°C for 3.5 hours under reflux. Then, toluene (20 ml) was added and dissolved and the mixture was then subjected to silica gel column chromatography. It was eluted by a developing solvent of hexane / ethyl acetate = 3/1 and a fraction containing an aimed product was concentrated by distilling off the solvent, to obtain 10.5 g (yield: 75%) of 2-methylindole-3-carboxaldehyde imine.
¹H-NMR spectra (chemical shift, ppm: in CDCl₃):
2.63 (3H, s), 7.15-7.42 (8H, m), 8.19 (1H, s), 8.45-8.48 (1H, m), 8.69 (1H, s).

2-methylindole-3-carboxaldehyde imine (100 mg, 4.5 mmol), methyl acrylate (0.194 g, 22.5 mmol) and diisopropyl ethyl amine (0.116 g, 9.0 mmol) were mixed in toluene (2 ml) and stirred at 80°C. Ethyl chloroformate (0.098g, 9.0 mmol) was dropped into the mixture. Thereafter, the reaction mixture was heated for one hour under reflux and allowed to cool to room temperature. Water was added and the mixture was extracted with toluene, and an organic layer was washed with an aqueous solution of sodium chloride and dried over sodium sulfate. The solvent was distilled off to obtain the crude product of N, 9-bis(ethoxycarbonyl)-N-phenyl-1-amino-1,2,3,4-tetrahydro-9H-carbazole-2-carboxylic acid methyl ester. It was subjected to silica gel column chromatography and eluted with a developing solvent of hexane / ethyl acetate = 5/1, and the fraction containing the aimed product was concentrated by distilling off the solvent, to obtain 150 mg (yield: 71.8% from imine) of N,9-bis(ethoxycarbonyl)-N-phenyl-1-amino-1,2,3,4-tetrahydro-9H-carbazole-3-carboxylic acid methyl ester.
¹H-NMR spectra (chemical shift, ppm: in CDCl₃):
1.15-1.25 (3H, m), 1.40-1.51 (3H, m), 1.88-2.19 (2H, m), 2.68-2.91 (2H, m), 2.95-3.11 (1H, m), 3.75 (3H, s), 4.15-4.32 (2H, m), 4.40-4.51 (2H, m), 6.25-6.35 (1H, d, J=7.9Hz), 6.75-6.88 (2H, m), 7.10-7.20 (2H, m), 7.22-7.38 (2H, m), 7.59-7.60 (1H, m), 8.10-8.20 (1H, m).

The thus obtained N,9-bis(ethoxycarbonyl-N-phenyl-1-amino-1,2,3,4-tetrahydro-9H-carbazole-3-carboxylic acid methyl ester was dissolved in methanol (10 ml), to which sodium methoxide (1 g in 28% methanol solution) was added. After stirring at room temperature for 2 hours, water was added to the reaction mixture and methanol was distilled off and then water was added to the residue, which was extracted with ethyl acetate. After washing the liquid extract with an aqueous solution of sodium chloride, it was dried over sodium sulfate. The solvent was distilled off and the residue was subjected to silica gel column chromatography, eluted with a hexane / ethyl acetate = 5/1 developing solvent and a fraction containing the aimed product was concentrated by distilling off the solvent, to obtain 80 mg (yield: 59.7% from imine) of 9-ethoxycarbonyl-1,2-dihydro-9H-carbazole-3-carboxylic acid methyl ester.
¹H-NMR spectra (chemical shift, ppm: in CDCl₃):
1.12-1.35 (3H, m), 2.43-2.68 (3H, m), 2.79-2,90 (1H, m), 3.75 (3H, s), 4.18-4.31 (2H, m), 6.77-6.89 (2H, m), 7.25-7.35 (1H, m), 7.58-7.65 (1H, d, J=6.7Hz), 7.77 (1H, s).

### Example 13

3-methylthiophene-2-aldehydimine (1 g, 5 mmol), dimethyl malonate (3.6 g, 25 mmol) and diisopropyl ethyl amine (1.28 g, 10 mmol) were mixed in toluene (4 ml) and stirred at 80°C. Ethyl chloroformate (1.07 g, 10 mmol) was dropped into the mixture. Thereafter, the reaction mixture was heated for one hour under reflux and allowed to cool to room temperature. Water was added and the mixture was extracted with toluene and an organic layer was washed with an aqueous solution of sodium chloride and dried over sodium sulfate. The solvent was distilled off to obtain the crude product of N-ethoxycarbonyl-N-phenyl-7-amino-4,5,6,7-tetrahydrobenzo[b]thiophene-5,6-dicarboxylic acid dimethyl ester. It was put to silica gel column chromatography and eluted with a developing solution of hexane / ethyl acetate = 3/1, and a fraction containing the aimed product was concentrated by distilling off the solvent, to obtain 1.2 g (yield: 57.7% from imine) of N-ethoxycarbonyl-N-phenyl-7-amino-4,5,6,7-tetrahydrobenzo [b]thiophene-5,6-dicarboxylic acid dimethyl ester.
¹H-NMR spectra (chemical shift, ppm: in CDCl₃):
0.90-1.30 (3H, m), 1.58-2.73 (joined 2H, m), 2.80-3.25 (2H, m), 3.50-3.80 (6H, m), 3,90-4.30 (2H, m), 5.10-6.30 (joined 1H, m), 6.60-6.95 (2H, m), 7.10-7.60 (5H, m).

The thus obtained N-ethoxycarbonyl-N-phenyl-7-amino-4,5,6,7-tetrahydrobenzo[b]thiophene-5,6-dicarboxylic acid dimethyl ester was dissolved in methanol (10 ml), to which sodium methoxide (1 g in 28% methanol solution) was added and stirred at room temperature for 2 hours. After adding water to the reaction mixture and distilling off methanol, water was added to the residue, which was extracted with ethyl acetate. The liquid extract was washed with an aqueous solution of sodium chloride and then dried over sodium sulfate. The solvent was distilled off and the residue was sujected to silica gel column chromatography and eluted with a developing solvent of hexane / ethyl acetate = 3/1, and the fraction containing an aimed product was concentrated by distilling off the solvent, to obtain 0.6 g (yield: 47.5% from imine) of 4,5-dihydrobenzo[b]thiophene-5,6-dicarboxylic acid dimethyl ester.
¹H-NMR spectra (chemical shift, ppm: in CDCl₃):
2.90-3.10 (1H, m), 3.40-3.58 (1H, m), 3.53 (3H, s), 3.83 (3H, m), 3.85-4.00 (1H, m), 6.85-6.98 (1H, d, J=4.9Hz), 7.27-7.40 (1H, d, J=4.9Hz), 7.67 (1H, s).

### Example 14

3-methylthiophene-2-aldehydimine (1 g, 5 mmol), acrylonitrile (1.35 g, 25 mmol) and diisopropyl ethyl amine (1.28 g, 10 mmol) were mixed in toluene (4 ml) and stirred at 80°C. Ethyl chloroformate (1.07 g, 10 mmol) was dropped into the mixture. Thereafter, the reaction mixture was heated for one hour under reflux and then allowed to cool to room temperature. Water was added and the mixture extracted with toluene, the organic layer was washed with an aqueous solution of sodium chloride and then dried over sodium sulfate. The solvent was distilled off to obtain the crude product of N-ethoxycarbonyl-N-phenyl-7-amino-4,5,6,7-tetrahydrobenzo[b]thiophene-6-nitrile. The resultant residue was purified by recrystallization (ethyl acetate / hexane) to obtain 0.65 g (yield: 40.0% from imine) of N-ethoxycarbonyl-N-phenyl-7-amino-4,5,6,7-tetrahydrobenzo[b]thiophene-6-nitrile.
¹H-NMR spectra (chemical shift, ppm: in CDCl₃):
1.00-1.30 (4H, m), 1.60 (1H, s), 1.70-1.89 (1H, m), 2.31-2.60 (2H, m), 3.11-3.27 (1H, m), 4.00-4.36 (2H, m), 6.25 (1H, s), 6.62-6.75 (1H, m), 6.90-7.11 (2H, s), 7.12-7.38 (3H, m).

The thus obtained N-ethoxycarbonyl-N-phenyl-7-amino-4,5,6,7-tetrahydrobenzo[b]thiophane-6-nitrile (100 mg) was dissolved in methanol (10 ml), to which sodium methoxide (1 g in 28% methanol solution) was added and stirred at 60°C for 2 hours. Then, water was added to the reaction mixture and, after distilling off the methanol, water was added to the residue, which was extracted with ethyl acetate. The liquid extract was washed with an aqueous solution of sodium chloride and then dried over sodium sulfate. The solvent was distilled off and the residue was subjected to silica gel column chromatography and eluted with a developing solvent of hexane / ethyl acetate = 3/1, and the fraction containing the aimed product was concentrated by distilling off the solvent, to obtain 40 mg (yield: 32.0% from imine) of 4,5,-dihydrobenzo[b]thiophene-6-nitrile.
¹H-NMR spectra (chemical shift, ppm: in CDCl₃):
2.49-2.70 (2H, m), 2.74-3.00 (2H, m), 6.80-6.95 (1H, m), 7.16 (1H, s), 7.27-7.41 (1H, m).

### Example 15

3-methylthiophene-2-aldehydimine (1 g, 5 mmol), methyl vinyl ketone (1.75 g, 25 mmol) and diisopropyl ethyl amine (1.28 g, 10 mmol) were mixed in toluene (4 ml) and stirred at 80°C. Ethyl chloroformate (1.07 g, 10 mmol) was dropped into the mixture. Thereafter, dropping, the reaction mixture was heated for one hour under reflux and then allowed to cool to room temperature. Water was added and the mixture was extracted with toluene and washed with an aqueous solution of sodium chloride and dried over sodium sulfate. The solvent was distilled off to obtain the crude product of N-ethoxycarbonyl-N-phenyl-7-amino-4,5,6,7-tetrahydrobenzo[b]thiophene-6-acetyl. The resultant residue was purified by recrystallization (ethyl acetate / hexane) to obtain 0.70 g (yield: 40% from imine) of N-ethoxycarbonyl-N-phenyl-7-amino-4,5,6,7-tetrahydrobenzo[b]thiophene-6-acetyl.
¹H-NMR spectra (chemical shift, ppm: in CDCl₃):
0.85-1.35 (4H, m), 1.50-1.90 (2H, m), 2.05-2.60 (4H, m), 2.90-3.08 (1H, m), 3.95-4.28 (2H, m), 5.90-6.77 (joined 3H, m), 6.90-7.31 (joined 5H, m).

To the thus obtained N-ethoxycarbonyl-N-phenyl-7-amino-4,5,6,7-tetrahydrobenzo[b]thiophene-6-acetyl (100 mg) was dissolved in methanol (10 ml), to which sodium methoxide (1 g in 28% methanol solution) was added and stirred at a room temperature for one hour. Then, water was added to the reaction mixture, methanol was distilled off and then water was added to the residue, which was extracted with ethyl acetate. The liquid extract was washed with an aqueous solution of sodium chloride and dried over sodium sulfate. The solvent was distilled off and the residue was subjected to silica gel column chromatography and eluted with a developing solvent of hexane / ethyl acetate = 10/1, and a fraction containing the aimed product was concentrated by distilling off the solvent, to obtain 40 mg (yield: 31.2% from imine) of 4,5-dihydrobenzo[b]thiophene-6-acetyl.
¹H-NMR spectra (chemical shift, ppm: in CDCl₃):
2.31 (3H, m), 2.40-2.85 (joined 4H, m), 6.71 (1H, m), 6.75-6.89 (1H, m), 6.90-7.01 (1H, m).

### Example 16

3-methylfuran-2-carboxaldehyde (10 g, 0.09 mol) was mixed with hexane (40 ml), to which aniline (9.6 ml, 0.11 mol) was added and stirred at room temperature for 30 minutes. Then, the reaction mixture was concentrated by distilling off the solvent and then subjected to silica gel column chromatography. It was eluted with a developing solvent of hexane / ethyl acetate = 20/1 and a fraction containing an aimed product was concentrated by distilling off the solvent, to obtain 5.2 g (yield: 31%) of 3-methylfuran-2-carboxaldehyde imine.
¹H-NMR spectra (chemical shift, ppm: in CDCl₃):
2.32 (3H, s), 6.63 (1H, d, J=4.9Hz), 7.05-7.25 (3H), 7.26-7.40 (3H), 8.32 (1H, s).

3-methylfuran-2-carboxaldehyde imine (1.5 g, 8.15 mmol), methyl acrylate (3.5 g, 40.7 mmol) and diisopropyl ethyl amine (2.1 g, 16.3 mmol) were mixed in toluene (6 ml) and stirred at 80°C. Ethyl chloroformate (1.77 g, 16.3 mmol) was dropped to the mixture. Thereafter, the reaction mixture was heated for one hour under reflux and allowed to cool to room temperature. Water was added and the mixture was extracted with toluene, and the organic layer was washed with an aqueous solution of sodium chloride and dried over sodium sulfate. The solvent was distilled off to obtain a crude product of N-ethoxycarbonyl-N-phenyl-7-amino-4,5,6,7-tetrahydrobenzo[b]furan-6-carboxylic acid methyl ester. It was subjected to silica gel chromatography and eluted with a developing solvent of hexane / ethyl acetate = 5/1, and the fraction containing the aimed product was concentrated by distilling off the solvent, to obtain 1.2 g (yield: 43.0% from imine) of N-ethoxycarbonyl-N-phenyl-7-amino-4,5,6,7-tetrahydrobenzo [b]furan-6-carboxylic acid methyl ester.
¹H-NMR spectra (chemical shift, ppm: in CDCl₃):
1.00-1.40 (3H, m), 1.65-2.10 (2H, m), 2.5-3.05 (joined 2H, m), 3.74 (3H, s), 3.95-4.30 (3H, m), 5.75-6.20 (joined 1H, m), 6.50-6.95 (2H, m), 7.00-7.50 (5H, m).

The thus obtained N-ethoxycarbonyl-N-phenyl-7-amino-4,5,6,7-tetrahydrobenzo[b]furan-6-carboxylic acid methyl ester was dissolved in methanol (10 ml), to which sodium methoxide (1 g in 28% methanol solution) was added. After stirring at 60°C for 2 hours, water was added to the reaction mixture and methanol was distilled off and then water was added to the residue, which was extracted with ethyl acetate. After washing the liquid extract with an aqueous solution of sodium chloride, it was dried over sodium sulfate. The solvent was distilled off and the residue was subjected to silica gel column chromatography, eluted with a developing solution of hexane / ethyl acetate = 9/1, and a fraction containing the aimed product was concentrated by distilling off the solvent, to obtain 0.5 g (yield: 34.7% from imine) of 4,5-dihydrobenzo[b]furan-6-carboxylic acid methyl ester.
¹H-NMR spectra (chemical shift, ppm: in CDCl₃): 2.51-2.80 (4H, m), 3.78 (3H, s), 6.33 (1H, s), 7.31-7.42 (2H, m).

## Claims

1. 1-amino-2-cyclohexene derivative represented by the following general formula (I): 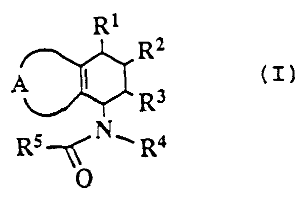 where A represents a bivalent organic group which may contain 1 to 3 oxygen atoms, nitrogen atoms and/or sulfur atoms, in which A may form a 5-membered ring, 6-membered ring, 7-membered ring or 8-membered ring together with two carbon atoms to be bonded, and the ring may form a condensed ring with one or a plurality of other rings; R¹ represents a hydrogen atom, an alkyl group, an alkenyl group, an aryl group or an aralkyl group, R² represents a hydrogen atom, an alkyl group, an alkenyl group, an aryl group, an aralkyl group, a cyano group or a group represented by the formula: -COR²¹, R²¹ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, R³ represents a cyano group, a nitro group or a group represented by the formula: -COR³¹, R³¹ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, R⁴ represents an alkyl group, an alkenyl group, an aryl group or an aralkyl group, R⁵ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, in which R² and R³¹ may join together to form a bivalent organic group possibly containing an oxygen atom or a nitrogen atom.

2. 1-amino-2-cyclohexene derivative as defined in claim 1, wherein the ring formed by A together with two carbon atoms to be joined is a thiophene ring.

3. 1-amino-2-cyclohexene derivative as defined in claim 1, wherein the ring formed by A together with two carbon atoms to be joined is an indole ring.

4. 1-amino-2-cyclohexene derivative as defined in claim 1, wherein the ring formed by A together with two carbon atoms to be joined is a furan ring.

5. 1-amino-2-cyclohexene derivative as defined in any one of claims 1 to 4, wherein R⁵ is an alkoxyl group, an aryloxyl group or an aralkyloxyl group.

6. 1-amino-2-cyclohexene derivative as defined in any one of claims 1 to 5, wherein R³ represents a group represented by the formula: -COR³¹, and R³¹ represents an alkoxyl group, an aryloxyl group or an aralkyloxyl group.

7. A tetrahydrobenzothiophene derivative or tetrahydrobenzofuran derivative represented by the following general formula (I-1): 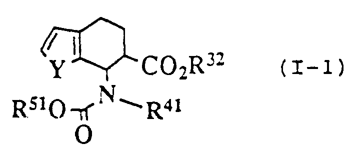 where each of R³², R⁴¹ and R⁵¹ represents an alkyl group, an aryl group or an aralkyl group and Y represents a sulfur atom or an oxygen atom.

8. A tetrahydrobenzothiophene derivative as defined in claim 7, wherein Y represents a sulfur atom.

9. A tetrahydrocarbazole derivative represented by the following general formula (I-2): 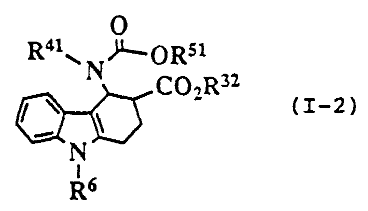 where each of R³², R⁴¹ and R⁵¹ represents an alkyl group, an aryl group or an aralkyl group, R⁶ represents an alkyl group, an aralkyl group, an acyl group, an alkoxycarbonyl group, an alkane sulfonyl group or an arene sulfonyl group.

10. A process for producing 1-amino-2-cyclohexene derivatives as defined in any one of claims 1 to 6 which comprises condensating an aldehyde represented by the general formula (II): 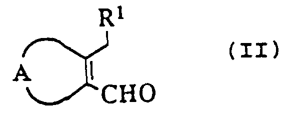 where A and R¹ are as defined above with a primary amine represented by the following general formula (III):
H₂NR⁴ (III)
where R⁴ is as defined above to obtain an imine represented by the following general formula (IV): 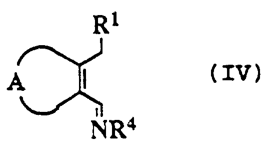 where A, R¹ and R⁴ are as defined above, and reacting the imine (IV) in the presence of a basic substance with a carbonylating agent represented by the following general formula (V): 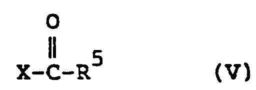 where R⁵ is as defined above and X represents a leaving group and an ethylene derivative represented by the following general formula (VI):
R²-CH=CH-R³ (VI)
where R² and R³ are as defined above.

11. A process for producing a 1-amino-2-cyclohexene derivative as defined in claim 10, wherein the carbonylating agent is chloroformate ester.

12. A process for producing a 1-amino-2-cyclohexene derivative as defined in any one of claims 10 to 11, whereinthe ethylene derivative is an acrylate ester.

13. A process for producing a 1-amino-2-cyclohexene derivative as defined in any one of claims 10, 11 or 12, wherein the aldehyde is 3-methylthiophene-2-aldehyde or 3-methylfuran-2-aldenyde represented by the following formula (VII): 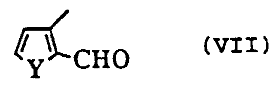 where Y represents a sulfur atom or an oxygen atom.

14. A process for producing a 1-amino-2-cyclohexene derivative as defined in claim 13, wherein Y represents a sulfur atom.

15. A process for producing a 1-amino-2-cyclohexene derivative as defined in any one of claims 10, 11 or 12, wherein the aldehyde is 2-methylindole-3-aldehyde represented by the following formula (VIII):

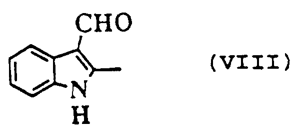

16. A process for producing 1,3-cyclohexadiene derivative represented by the following general formula (IX): 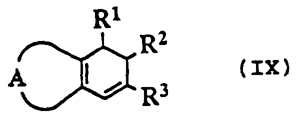 where A represents a bivalent organic group which may contain 1 to 3 oxygen atoms, nitrogen atoms and/or sulfur atoms, in which A may form a 5-membered ring, 6-membered ring, 7-membered ring or 8-membered ring together with two carbon atoms to be bonded, and the ring may form a condensed ring with one or a plurality of other rings R¹ represents a hydrogen atom, an alkyl group, an alkenyl group, an aryl group or an aralkyl group, R² represents a hydrogen atom, an alkyl group, an alkenyl group, an aryl group, an aralkyl group, a cyano group or a group represented by the formula: -COR²¹, R²¹ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, and R³ represents a cyano group, a nitro group or a group represented by the formula: -COR³¹, R³¹ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, in which R² and R³¹ may join together to form a bivalent organic group possibly containing an oxygen atom or a nitrogen atom, which comprises subjecting a 1-amino-2-cyclohexene derivative represented by the following general formula (I) : 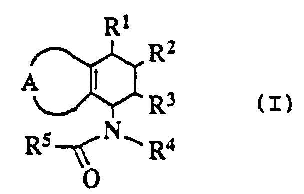 where A, R¹, R² and R³ are as defined above, R⁴ represents an alkyl group, an alkenyl group, an aryl group or an aralkyl group, and R⁵ represents a hydrogen atom, an alkyl group, an alkenyl group, an aryl group, an aralkyl group, an alkoxyl group, an alkenyloxyl group, an aryloxyl group, an aralkyloxyl group or an amino group which may have a substituent, to an elimination reaction using a basic substance.

17. A process for producing a 1,3-cyclohexadiene derivative as defined in claim 16, wherein the ring formed by A together with two carbon atoms to be joined is a thiophene ring.

18. A process for producing a 1,3-cyclohexadiene derivative as defined in claim 16, wherein the ring formed by A together with two carbon atoms to be joined is an indole ring.

19. A process for producing a 1,3-cyclohexadiene derivative as defined in claim 16, wherein the ring formed by A together with two carbon atoms to be joined is an furan ring.

20. A process for producing a dihydrobenzothiophene carboxylic acid derivative or dihydrobenzofuran carboxylic acid derivative represented by the following general formula (IX-1): 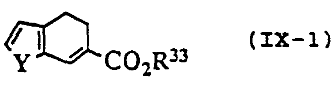 where Y represents a sulfur atom or oxygen atom and R³³ represents a hydrogen atom, an alkyl group, aryl group, or aralkyl group, in which a tetrahydrobenzothiophene derivative or tetrahydrobenzofuran derivative represented by the following general formula (I-1): 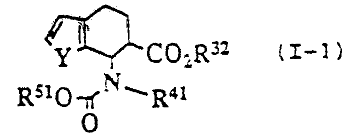 where each of R³², R⁴¹ and R⁵¹ represents an alkyl group or an aralkyl group and Y is as defined above, is subjected to elimination reaction with a basic substance and, if necessary, to a hydrolyzing reaction.

21. A process for producing the dihydrobenzothiophene carboxylic acid derivative as defined in claim 20, wherein Y represents a sulfur atom.

22. A process for producing a dihydrocarbazole carboxylic acid derivative represented by the following general formula (IX-2): 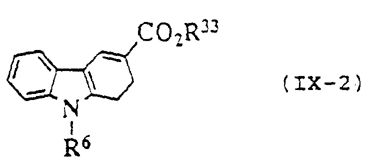 where R³³ represents a hydrogen atom, an alkyl group, aryl group or aralkyl group and R⁶ represents an alkyl group, aralkyl group, acyl group, alkoxycarbonyl group, alkane sulfonyl group or arene sulfonyl group in which a tetrahydrocarbazole derivative represented by the following general formula (I-2): 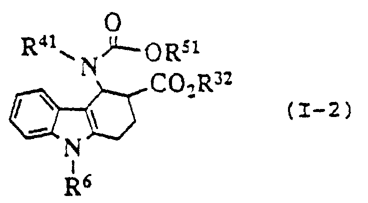 where each of R³², R⁴¹ and R⁵¹ represents an alkyl group, an aryl group, or an aralkyl group and R⁶ is as defined above, is subjected to elimination reaction with a basic substance and, if necessary, to hydrolyzing reaction.

## Patentansprüche

1. 1-Amino-2-cyclohexen-Derivat, dargestellt durch die folgende allgemeine Formel (I): 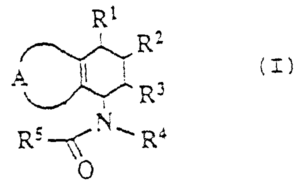 worin A einen bivalenten organischen Rest darstellt, welcher 1 bis 3 Sauerstoff-, Stickstoff- und/oder Schwefelatome enthalten kann, worin A einen 5-, 6-, 7- oder 8-gliedrigen Ring zusammen mit 2 zu bindenden Kohlenstoffatomen bilden kann und der Ring einen kondensierten Ring mit einem oder einer Vielzahl von anderen Ringen bilden kann; R¹ ein Wasserstoffatom, einen Alkyl-, Alkenyl-, Aryl- oder Aralkylrest darstellt, R² ein Wasserstoffatom, einen Alkyl-, Alkenyl-, Aryl-, Aralkylrest, eine Cyanogruppe oder einen Rest, dargestellt durch die Formel -COR²¹, darstellt, R²¹ ein Wasserstoffatom, einen Alkyl-, Alkenyl-, Aryl-, Aralkyl-, Alkoxy-, Alkenyloxy-, Aryloxy-, Aralkyloxyrest oder eine Aminogruppe, welche substituiert sein kann, darstellt, R³ eine Cyano-, Nitrogruppe oder einen Rest, dargestellt durch die Formel -COR³¹, darstellt, R³¹ ein Wasserstoffatom, einen Alkyl-, Alkenyl-, Aryl-, Aralkyl-, Alkoxy-, Alkenyloxy-, Aryloxy-, Aralkyloxyrest oder eine Aminogruppe, welche substituiert sein kann, darstellt, R⁴ einen Alkyl-, Alkenyl-, Aryl- oder Aralkylrest darstellt, R⁵ ein Wasserstoffatom, einen Alkyl-, Alkenyl-, Aryl-, Aralkyl-, Alkoxy-, Alkenyloxy-, Aryloxy-, Aralkyloxyrest oder eine Aminogruppe, welche substituiert sein kann, darstellt, worin R² und R³¹ zusammen einen bivalenten organischen Rest bilden können, welcher ein Sauerstoff- oder Stickstoffatom enthalten kann.

2. 1-Amino-2-cyclohexen-Derivat, wie in Anspruch 1 definiert, worin der Ring, welcher durch A zusammen mit 2 zu bindenden Kohlenstoffatomen gebildet wird, ein Thiophenring ist.

3. 1-Amino-2-cyclohexen-Derivat, wie in Anspruch 1 definiert, worin der Ring, welcher durch A zusammen mit 2 zu bindenden Kohlenstoffatomen gebildet wird, ein Indolring ist.

4. 1-Amino-2-cyclohexen-Derivat, wie in Anspruch 1 definiert, worin der Ring, welcher durch A zusammen mit 2 zu bindenden Kohlenstoffatomen gebildet wird, ein Furanring ist.

5. 1-Amino-2-cyclohexen-Derivat, wie in einem der Ansprüche 1 bis 4 definiert, worin R⁵ ein Alkoxy-, Aryloxy- oder Aralkyloxyrest ist.

6. 1-Amino-2-cyclohexen-Derivat, wie in einem der Ansprüche 1 bis 5 definiert, worin R³ einen Rest, dargestellt durch die Formel -COR³¹, darstellt und R³¹ einen Alkoxy-, Aryloxy- oder Aralkyloxyrest darstellt.

7. Tetrahydrobenzothiophen-Derivat oder Tetrahydrobenzofuran-Derivat, dargestellt durch die folgende allgemeine Formel (I-1): 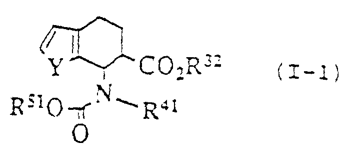 worin jeder der Reste R³², R⁴¹ und R⁵¹ einen Alkyl-, Aryl- oder Aralkylrest darstellt und Y ein Schwefel- oder Sauerstoffatom darstellt.

8. Tetrahydrobenzothiophen-Derivat, wie in Anspruch 7 definiert, worin Y ein Schwefelatom darstellt.

9. Tetrahydrocarbazol-Derivat, dargestellt durch die folgende allgemeine Formel (I-2): 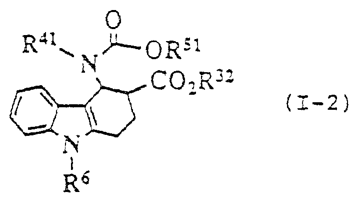 worin jeder der Reste R³², R⁴¹ und R⁵¹ einen Alkyl-, Aryl- oder Aralkylrest darstellt, R⁶ einen Alkyl-, Aralkyl-, Acyl-, Alkoxycarbonyl-, Alkansulfonyl-oder Arensulfonylrest darstellt.

10. Verfahren zur Herstellung von 1-Amino-2-cyclohexen-Derivaten, wie in einem der Ansprüche 1 bis 6 definiert, umfassend die Kondensation eines Aldehyds, dargestellt durch die allgemeine Formel (II): 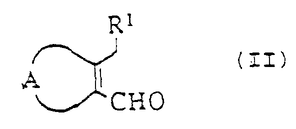 worin A und R¹ wie vorstehend definiert sind, mit einem primären Amin, dargestellt durch die folgende allgemeine Formel (III):
H₂NR⁴ (III)
worin R⁴ wie vorstehend definiert ist, um ein lmin zu erhalten, dargestellt durch die folgende allgemeine Formel (IV): 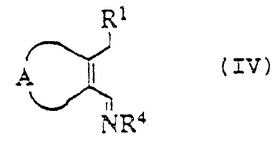 worin A, R¹ und R⁴ wie vorstehend definiert sind, und Umsetzen des lmins (IV) in Gegenwart eines basischen Stoffes mit einem carbonylierenden Agens, dargestellt durch die folgende allgemeine Formel (V): 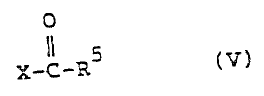 worin R⁵ wie vorstehend definiert ist und X eine Abgangsgruppe darstellt, und einem Ethylen-Derivat, dargestellt durch die folgende allgemeine Formel (VI):
R²-CH=CH-R³ (VI)
worin R² und R³ wie vorstehend definiert sind.

11. Verfahren zur Herstellung eines 1-Amino-2-cyclohexen-Derivats, wie in Anspruch 10 definiert, worin das carbonylierende Agens ein Chlorformatester ist.

12. Verfahren zur Herstellung eines 1-Amino-2-cyclohexen-Derivats, wie in einem der Ansprüche 10 bis 11 definiert, worin das Ethylen-Derivat ein Acrylatester ist.

13. Verfahren zur Herstellung eines 1-Amino-2-cyclohexen-Derivats, wie in einem der Ansprüche 10, 11 oder 12 definiert, worin der Aldehyd 3-Methylthiophen-2-aldehyd oder 3-Methylfuran-2-aldehyd ist, dargestellt durch die folgende Formel (VII): 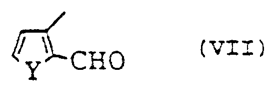 worin Y ein Schwefel- oder Sauerstoffatom darstellt.

14. Verfahren zur Herstellung eines 1-Amino-2-cyclohexen-Derivats, wie in Anspruch 13 definiert, wobei Y ein Schwefelatom darstellt.

15. Verfahren zur Herstellung eines 1-Amino-2-cyclohexen-Derivats, wie in einem der Ansprüche 10, 11 oder 12 definiert, wobei der Aldehyd 2-Methylindol-3-aldehyd ist, dargestellt durch die folgende Formel (VIII):

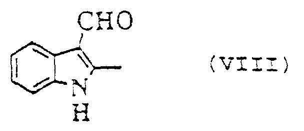

16. Verfahren zur Herstellung eines 1,3-Cyclohexadien-Derivats, dargestellt durch die folgende allgemeine Formel (IX): 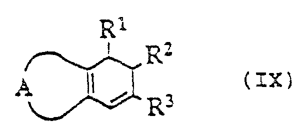 worin A einen bivalenten organischen Rest darstellt, welcher 1 bis 3 Sauerstoff-, Stickstoff- und/oder Schwefelatome enthalten kann, worin A einen 5-, 6-, 7- oder 8-gliedrigen Ring mit 2 zu bindenden Kohlenstoffatomen bilden kann und der Ring einen kondensierten Ring mit einem Ring oder einer Vielzahl von anderen Ringen bilden kann; R¹ ein Wasserstoffatom, einen Alkyl-, Alkenyl-, Aryl- oder Aralkylrest darstellt, R² ein Wasserstoffatom, einen Alkyl-, Alkenyl-, Aryl-, Aralkylrest, eine Cyanogruppe oder einen Rest, dargesteift durch die Formel -COR²¹, darstellt, R²¹ ein Wasserstoffatom, einen Alkyl-, Alkenyl-, Aryl-, Aralkyl-, Alkoxy-, Alkenyloxy-, Aryloxy-, Aralkyloxyrest oder eine Aminogruppe, welche substituiert sein kann, darstellt, und R³ eine Cyano-, Nitrogruppe oder einen Rest, dargestellt durch die Formel -COR³¹, darstellt, R³¹ ein Wasserstoffatom, einen Alkyl-, Alkenyl-, Aryl-, Aralkyl-, Alkoxy-, Alkenyloxy-, Aryloxy-, Aralkyloxyrest oder eine Aminogruppe, welche substituiert sein kann, darstellt, worin R² und R³¹ zusammen einen bivalenten organischen Rest bilden können, welcher ein Sauerstoff- oder Stickstoffatom enthalten kann, umfassend das Unterwerfen eines 1-Amino-2-cyclohexen-Derivats, dargestellt durch die folgende allgemeine Formel (I): 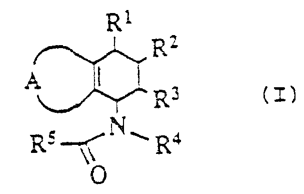 worin A, R¹, R² und R³ wie vorstehend definiert sind, R⁴ einen Alkyl-, Alkenyl-, Aryl- oder Aralkylrest darstellt und R⁵ ein Wasserstoffatom, einen Alkyl-, Alkenyl-, Aryl-, Aralkyl-, Alkoxy-, Alkenyloxy-, Aryloxy-, Aralkyloxylrest oder eine Aminogruppe, welche substituiert sein kann, darstellt, einer Eliminierungsreaktion unter Verwendung eines basischen Stoffes.

17. Verfahren zur Herstellung eines 1,3-Cyclohexadien-Derivats, wie in Anspruch 16 definiert, worin der Ring, welcher von A zusammen mit 2 zu bindenden Kohlenstoffatomen gebildet wird, ein Thiophenring ist.

18. Verfahren zur Herstellung eines 1,3-Cyclohexadien-Derivats, wie in Anspruch 16 definiert, worin der Ring, welcher von A zusammen mit 2 zu bindenden Kohlenstoffatomen gebildet wird, ein Indolring ist.

19. Verfahren zur Herstellung eines 1,3-Cyclohexadien-Derivats, wie in Anspruch 16 definiert, worin der Ring, welcher von A zusammen mit 2 zu bindenden Kohlenstoffatomen gebildet wird, ein Furanring ist.

20. Verfahren zur Herstellung eines Dihydrobenzothiophencarbonsäure-Derivats oder Dihydrobenzofurancarbonsäure-Derivats, dargestellt durch die folgende allgemeine Formel (IX-1): 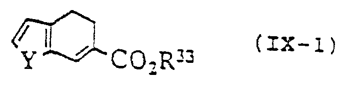 worin Y ein Schwefel- oder Sauerstoffatom darstellt und R³³ ein Wasserstoffatom, einen Alkyl-, Aryl- oder Aralkylrest darstellt, in welchem ein Tetrahydrobenzothiophen-Derivat oder Tetrahydrobenzofuran-Derivat, dargestellt durch die folgende allgemeine Formel (I-1): 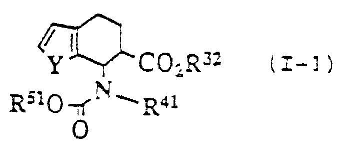 worin jeder der Reste R³², R⁴¹ und R⁵¹ einen Alkyl- oder Aralkylrest darstellt und Y wie vorstehend definiert ist, einer Eiiminierungsreaktion mit einem basischen Stoff und, falls nötig, einer Hydrolysereaktion unterworfen wird.

21. Verfahren zur Herstellung eines Dihydrobenzothiophencarbonsäure-Derivats, wie in Anspruch 20 definiert, worin Y ein Schwefelatom darstellt.

22. Verfahren zur Herstellung eines Dihydrocarbazolcarbonsäure-Derivats, dargestellt durch die folgende allgemeine Formel (IX-2): 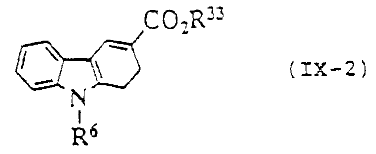 worin R³³ ein Wasserstoffatom, einen Alkyl-, Aryl- oder einen Aralkylrest darstellt und R⁶ einen Alkyl-, Aralkyl-, Acyl-, Alkoxycarbonyi-, Alkansulfonyl-oder Arensulfonylrest darstellt, worin ein Tetrahydrocarbazol-Derivat, dargestellt durch die folgende allgemeine Formel (I-2): 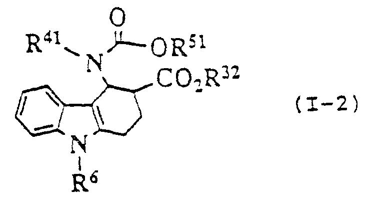 worin jeder der Reste R³², R⁴¹ und R⁵¹ einen Alkyl-, Aryl- oder Aralkylrest darstellt und R⁶ wie vorstehend definiert ist, einer Eliminierungsreaktion mit einem basischen Stoff und, falls nötig, einer Hydrolysereaktion unterworfen wird.

## Revendications

1. Dérivé de 1-amino-2-cyclohexène représenté par la formule générale (I) suivante : 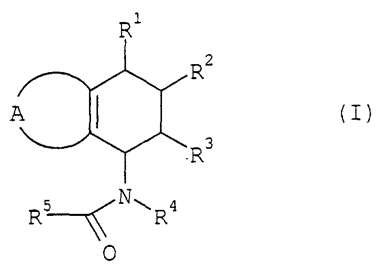 où A représente un groupe organique divalent qui peut contenir 1 à 3 atomes d'oxygène, atomes d'azote et/ou atomes de soufre, où A peut former un cycle à 5-chaînons, un cycle à 6 chaînons, un cycle à 7 chaînons ou un cycle à 8 chaînons avec deux atomes de carbone destinés à être liés, et le cycle peut former un cycle condensé avec un autre cycle ou une pluralité d'autres cycles ; R¹ représente un atome d'hydrogène, un groupe alkyle, un groupe alcényle, un groupe aryle ou un groupe aralkyle, R² représente un atome d'hydrogène, un groupe alkyle, un groupe alcényle, un groupe aryle, un groupe aralkyle, un groupe cyano ou un groupe représenté par la formule : -COR²¹, R²¹ représente un atome d'hydrogène, un groupe alkyle, un groupe alcényle, un groupe aryle, un groupe aralkyle, un groupe alcoxy, un groupe alcényloxy, un groupe aryloxy, un groupe aralkyloxy ou un groupe amino qui peut avoir un substituant, R³ représente un groupe cyano, un groupe nitro ou un groupe représenté par la formule : -COR³¹, R³¹ représente un atome d'hydrogène, un groupe alkyle, un groupe alcényle, un groupe aryle, un groupe aralkyle, un groupe alcoxy, un groupe alcényloxy, un groupe aryloxy, un groupe aralkyloxy ou un groupe amino qui peut avoir un substituant, R⁴ représente un groupe alkyle, un groupe alcényle, un groupe aryle ou un groupe aralkyle, R⁵ représente un atome d'hydrogène, un groupe alkyle, un groupe alcényle, un groupe aryle, un groupe aralkyle, un groupe alcoxy, un groupe alcényloxy, un groupe aryloxy, un groupe aralkyloxy ou un groupe amino qui peut avoir un substituant, où R² et R³¹ peuvent être liés pour former un groupe organique divalent contenant éventuellement un atome d'oxygène ou un atome d'azote.

2. Dérivé de 1-amino-2-cyclohexène selon la revendication 1 où le cycle formé par A avec deux atomes de carbone destinés à être liés est un cycle thiophène.

3. Dérivé de 1-amino-2-cyclohexène selon la revendication 1 où le cycle formé par A avec deux atomes de carbone destinés à être liés est un cycle indole.

4. Dérivé de 1-amino-2-cyclohexène selon la revendication 1 où le cycle formé par A avec deux atomes de carbone destinés à être liés est un cycle furane.

5. Dérivé de 1-amino-2-cyclohexène selon l'une quelconque des revendications 1 à 4 où R⁵ est un groupe alcoxy, un groupe aryloxy ou un groupe aralkyloxy.

6. Dérivé de 1-amino-2-cyclohexène selon l'une quelconque des revendications 1 à 5 où R³ représente un groupe représenté par la formule : -COR³¹, R³¹ représente un groupe alcoxy, un groupe aryloxy ou un groupe aralkyloxy.

7. Dérivé de tétrahydrobenzothiophène ou dérivé de tétrahydrobenzofurane représenté par la formule générale (I-1) suivante : 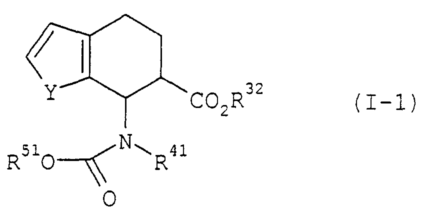 où R³², R⁴¹ et R⁵¹ représentent chacun un groupe alkyle, un groupe aryle ou un groupe aralkyle et Y représente un atome de soufre ou un atome d'oxygène.

8. Dérivé de tétrahydrobenzothiophène selon la revendication 7 où Y représente un atome de soufre.

9. Dérivé de tétrahydrocarbazole représenté par la formule générale (I-2) suivante : 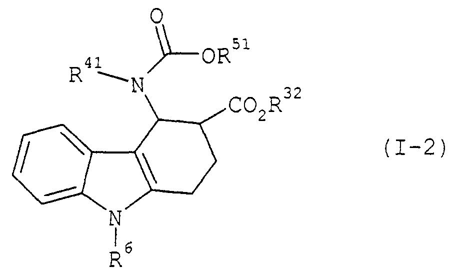 où R³², R⁴¹ et R⁵¹ représentent chacun un groupe alkyle, un groupe aryle ou un groupe aralkyle, R⁶ représente un groupe alkyle, un groupe aralkyle, un groupe acyle, un groupe alcoxycarbonyle, un groupe alcanesulfonyle ou un groupe arènesulfonyle.

10. Procédé pour produire des dérivés de 1-amino-2-cyclohexène selon l'une quelconque des revendications 1 à 6 qui comprend la condensation d'un aldéhyde représenté par la formule générale (II) : 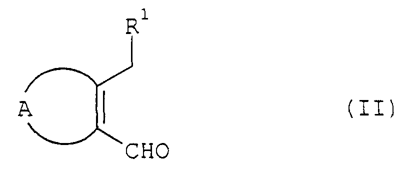 où A et R¹ sont tels que définis ci-dessus avec une amine primaire représentée par la formule générale (III) suivante :
H₂NR⁴ (III)
où R⁴ est tel que défini ci-dessus pour obtenir une imine représentée par la formule générale (IV) suivante : 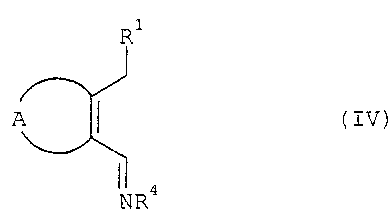 où A, R¹ et R⁴ sont tels que définis ci-dessus, et la réaction de l'imine (IV) en présence d'une substance basique avec un agent carbonylant représenté par la formule générale (V) suivante : 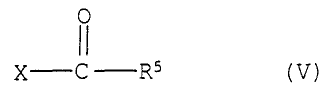 où R⁵ est tel que défini ci-dessus et X représente un groupe partant et un dérivé de l'éthylène représenté par la formule générale (VI) suivante :
R²-CH=CH-R³ (VI)
où R² et R³ sont tels que définis ci-dessus.

11. Procédé pour produire un dérivé de 1-amino-2-cyclohexène selon la revendication 10 où l'agent carbonylant est un ester chloroformiate.

12. Procédé pour produire un dérivé de 1-amino-2-cyclohexène selon l'une quelconque des revendications 10 à 11 où le dérivé de l'éthylène est un ester acrylate.

13. Procédé pour produire un dérivé de 1-amino-2-cyclohexène selon l'une quelconque des revendications 10, 11 ou 12 où l'aldéhyde est le 3-méthylthiophène-2-aldéhyde ou le 3-méthylfurane-2-aldéhyde représenté par la formule (VII) suivante : 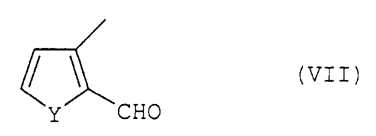 où Y représente un atome de soufre ou un atome d'oxygène.

14. Procédé pour produire un dérivé de 1-amino-2-cyclohexène selon la revendication 13 où Y représente un atome de soufre.

15. Procédé pour produire un dérivé de 1-amino-2-cyclohexène selon l'une quelconque des revendications 10, 11 ou 12 où l'aldéhyde est le 2-méthylindole-3-aldéhyde représenté par la formule (VIII) suivante :

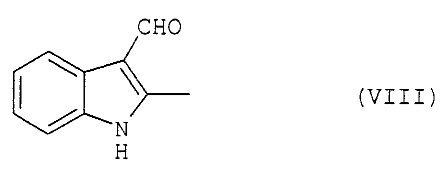

16. Procédé pour produire un dérivé de 1,3-cyclohexadiène représenté par la formule générale (IX) suivante : 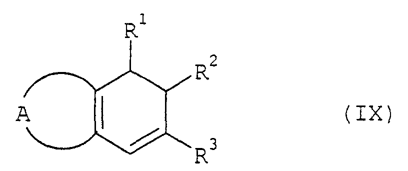 où A représente un groupe organique divalent qui peut contenir 1 à 3 atomes d'oxygène, atomes d'azote et/ou atomes de soufre, où A peut former un cycle à 5 chaînons, un cycle à 6 chaînons, un cycle à 7 chaînons ou un cycle à 8 chaînons avec deux atomes de carbone destinés à être liés, et le cycle peut former un cycle condensé avec un autre cycle ou une pluralité d'autres cycles ; R¹ représente un atome d'hydrogène, un groupe alkyle, un groupe alcényle, un groupe aryle ou un groupe aralkyle, R² représente un atome d'hydrogène, un groupe alkyle, un groupe alcényle, un groupe aryle, un groupe aralkyle, un groupe cyano ou un groupe représenté par la formule : -COR²¹, R²¹ représente un atome d'hydrogène, un groupe alkyle, un groupe alcényle, un groupe aryle, un groupe aralkyle, un groupe alcoxy, un groupe alcényloxy, un groupe aryloxy, un groupe aralkyloxy ou un groupe amino qui peut avoir un substituant, et R³ représente un groupe cyano, un groupe nitro ou un groupe représenté par la formule : - COR³¹, R³¹ représente un atome d'hydrogène, un groupe alkyle, un groupe alcényle, un groupe aryle, un groupe aralkyle, un groupe alcoxy, un groupe alcényloxy, un groupe aryloxy, un groupe aralkyloxy ou un groupe amino qui peut avoir un substituant, où R² et R³¹ peuvent être liés pour former un groupe organique divalent contenant éventuellement un atome d'oxygène ou un atome d'azote, qui comprend la soumission d'un dérivé de 1-amino-2-cyclohexène représenté par la formule générale (I) suivante : 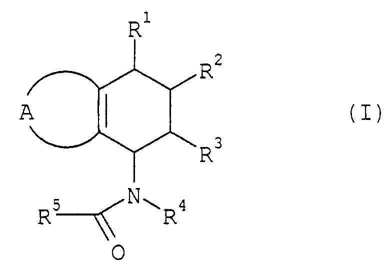 où A, R¹, R² et R³ sont tels que définis ci-dessus, R⁴ représente un groupe alkyle, un groupe alcényle, un groupe aryle ou un groupe aralkyle, et R⁵ représente un atome d'hydrogène, un groupe alkyle, un groupe alcényle, un groupe aryle, un groupe aralkyle, un groupe alcoxy, un groupe alcényloxy, un groupe aryloxy, un groupe aralkyloxy ou un groupe amino qui peut avoir un substituant, à une réaction d'élimination au moyen d'une substance basique.

17. Procédé pour produire un dérivé de 1,3-cyclohexadiène selon la revendication 16 où le cycle formé par A avec deux atomes de carbone destinés à être joints est un cycle thiophène.

18. Procédé pour produire un dérivé de 1,3-cyclohexadiène selon la revendication 16 où le cycle formé par A avec deux atomes de carbone destinés à être liés est un cycle indole.

19. Procédé pour produire un dérivé de 1,3-cyclohexadiène selon la revendication 16 où le cycle formé par A avec deux atomes de carbone destinés à être liés est un cycle furane.

20. Procédé pour produire un dérivé d'acide dihydrobenzothiophènecarboxylique ou un dérivé d'acide dihydrobenzofuranecarboxylique représenté par la formule générale (IX-1) suivante : 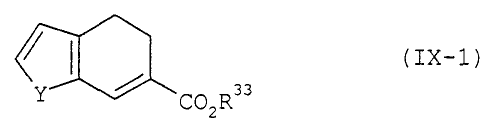 où Y représente un atome de soufre ou un atome d'oxygène et R³³ représente un atome d'hydrogène, un groupe alkyle, un groupe aryle ou un groupe aralkyle, où un dérivé de tétrahydrobenzothiophène ou un dérivé de tétrahydrobenzofurane représenté par la formule générale (I-1) suivante : 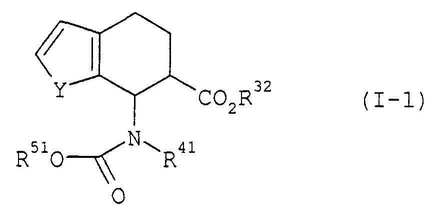 où R³², R⁴¹ et R⁵¹ représentent chacun un groupe alkyle ou un groupe aralkyle et Y est tel que défini ci-dessus est soumis à une réaction d'élimination avec une substance basique et, si nécessaire, à une réaction d'hydrolyse.

21. Procédé pour produire le dérivé d'acide dihydrobenzothiophènecarboxylique selon la revendication 20 où Y représente un atome de soufre.

22. Procédé pour produire un dérivé d'acide dihydrocarbazolecarboxylique représenté par la formule générale (IX-2) suivante : 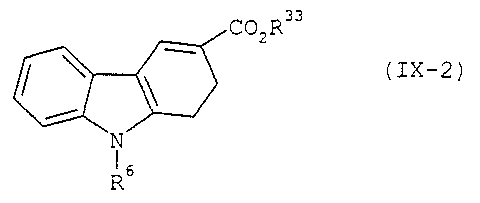 où R³³ représente un atome d'hydrogène, un groupe alkyle, un groupe aryle ou un groupe aralkyle et R⁶ représente un groupe alkyle, un groupe aralkyle, un groupe acyle, un groupe alcoxycarbonyle, un groupe alcanesulfonyle ou un groupe arènesulfonyle où un dérivé de tétrahydrocarbazole représenté par la formule générale (I-2) suivante : 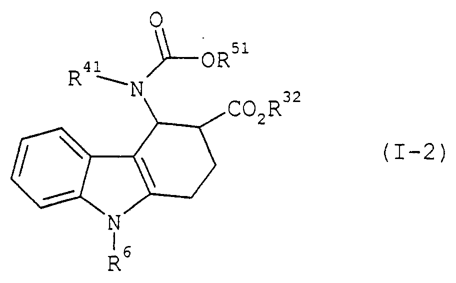 où R³², R⁴¹ et R⁵¹ représentent chacun un groupe alkyle, un groupe aryle ou un groupe aralkyle et R⁶ est tel que défini ci-dessus, est soumis à une réaction d'élimination avec une substance basique et, si nécessaire, à une réaction d'hydrolyse.
